# EUROPEAN PATENT APPLICATION

(11) **EP 4 575 483 A1**
(43) Date of publication of application: **25.06.2025**
(21) Application number: 23219755.8
(22) Date of filing: 22.12.2023
(51) Int. Cl.: G01N 27/27

(54) **ELECTROCHEMICAL SYSTEM FOR TESTING SAMPLE MATERIALS FOR ELECTROCATALYTIC REACTIONS**

(71) Applicant: VITO NV, 2400 Mol (BE)
(72) Inventor: CHEN, Zhiyuan, 2400 Mol (BE); VAN HOUTVEN, Diane, 2400 Mol (BE); BOUWMAN, Bert, 2400 Mol (BE); VANHOOF, Filip, 2400 Mol (BE); BIRDJA, Yuvraj Y., 2400 Mol (BE)
(74) Representative: Arnold & Siedsma

(57) **Abstract**

System for testing sample materials for electrocatalytic reactions, said system comprising two separate components consisting of a sample holder for holding the sample materials to be tested, and a sampling head for testing the sample materials, in particular in an automated manner for high throughput. The system has three électrodes, a working électrode, a référence électrode and a counter électrode. The system further includes a displacement arrangement for moving the sampling sample holder and/or the sampling head relative to each other and into a testing position.

## Description

The present invention relates a system for testing sample materials for electrocatalytic reactions, in particular to review the presence of desired electrochemical catalytic characteristics of said sample materials.

### Background

The industry strongly relies on catalysts for reactions and processes, in particular within the energy sector, pharmaceutical industry, and bulk chemical synthesis. Improvements in catalyst activity and selectivity favor the (energy) efficiency and can substantially decrease the operational costs of the process. Even a small improvement in the performance of the catalyst, may have significant cost benefits, see for example the production of ethylene, ammonia, ethylene oxide, methanol, urea. Further, electrochemical technologies are increasing in popularity in view of electrification of the chemical industry and the energy transition as alternative to fossil fuel-based processes. The electrochemical technologies having mature electrochemical processes are currently for chlorine production, fuel cell technologies and hydrogen production. However, there are many other processes currently still at lower TRLs with favorable prospects such as CO2 electroreduction, ammonia synthesis, and electro-organic synthesis, in which electrocatalysis plays an important role. These require electrochemical catalysts for which more testing and optimization is required.

To provide improvements within the electrochemical technologies, there is a need in understanding how the catalysts work. In essence, to get a good understanding, testing of the electrochemical catalysts is essential, in particular for both scientific understanding and practical applications, driving advancements in energy efficiency, environmental sustainability, and technological innovation.

However, current testing techniques are rather slow and burdensome posing hardship on operators when one has to test a high number of samples. Therefore, there is a need for an improved technique to facilitate testing of electrochemical reactions, in particular to get a good understanding on how the catalysts work.

### Summary of aspects of the invention

Hence, an object of embodiments of the present invention is to provide a technique for testing electrochemical reactions at a higher throughput, more specifically to provide a technique that can handle the testing of a large numbers of potential catalysts, in particular in order to test potential electrochemical catalysts, more in particular at a high throughput and in a reliable manner whereby accurate measurements can be achieved.

Thereto, a first aspect of the present invention provides a system for testing sample materials for electrocatalytic reactions, said system comprising two separate components consisting of a sample holder for holding the sample materials to be tested, and a sampling head for testing the sample materials, said sampling head comprising a sampling chamber separated from a second chamber via a membrane separator.

In particular, the system has three electrodes (i.e. an electrochemical cell with a three electrode configuration), namely, the sample holder is configured to operate as a working electrode, the sampling chamber comprises a reference electrode and the second chamber comprises a counter electrode. More in particular, the system further includes a displacement arrangement for moving the sampling head and/or sample holder relative to each other and into a testing position.

Insights of the inventors have provided that the three-electrode system provides for a better control, accuracy, and flexibility in studying electrochemical reactions. By using a reference electrode to control the potential and a separate counter electrode to facilitate the current flow, it minimizes interference between the potential control and the electrochemical processes happening at the working electrode. In contrast, a two-electrode setup typically combines the functions of the reference and counter electrodes, leading to interference and inaccuracies in measurements, this is not desired in situations where precise control is essential. Thus, the three-electrode system provides better control, accuracy, and flexibility in studying electrochemical reactions by decoupling the potential control and current measurement, making it advantageous to test sample materials, preferably potential electrochemical catalysts, in a reliable manner.

Further, insights of the inventors have provided that electrochemical reactions can be tested in the sampling chamber at an overall higher throughput by way of the displacement arrangement that is included within the system. Namely, the sampling head and/or sample holder can be moved into a testing position without the need of lab operators or with other words, without the need of people input for putting the sample materials into testing position.

Preferably, the displacement arrangement comprises a positioning stage, such as a movable table and/or rotatable table, configured to position the sample holder and/or comprises one or more actuators to position the sampling head. Preferably the actuator(s) are configured to operate a robotic system, e.g. a robotic arm, on which the sampling head is arranged. More in particular, the actuator(s) are preferably arranged to move the sampling head along an X direction and/or Y direction and preferably a Z direction as well, with respect to the sample holder.

Preferably, the sampling chamber comprises a sample opening configured to be placed on or over a sample on the sample holder. In this manner, sample materials can be tested in an automated manner. In a "placed on" condition, edges of the opening may be placed on a sample, e.g. a sample coated on a wafer held by the sample holder. The "placed over" condition, refers to having edges of the opening circumvent the sample such that said sample is surrounded by the opening. The wafer can function as a removable sample holding structure. The ability to remove the sample holding structure allows for easy loading and unloading of samples.

Preferably, the sampling head comprises a sealing ring arranged to seal off the sampling chamber when the sample opening is placed on or over a sample on the sample holder. In this way, testing and accuracy can be improved as the testing conditions within the sampling chamber are shielded from outside factors. More in particular, the sampling chamber can be filled with an electrolyte composition and said electrolyte composition is better held within the sampling chamber of the sampling head by way of the sealing ring sealing off the sampling chamber. Preferably, the sealing ring is an O-ring or an X-ring, more preferably an X-ring. The X ring has been found to better perform in dynamic situations. This is because the X shape creates at least four sealing surfaces. Preferably, the sealing ring is an X ring and/or is made of EPDM, NBR, FKM (viton), Silicone. EPDM has been found to be performing well in case of contact with water (steam), mild chemicals, acids, synthetic oils, alkalis. NBR (nitrile butadiene rubber) has been found to be working well with Water, Petroleum Oils & Fluids, and Hydraulic Fluids. FKM has been found to be working well with Petroleum base oils and fluids, some phosphate ester base fluids, silicone and silicate ester base lubricants, acids and halogenated hydrocarbons. Silicone has been found to be working well with Water, dry heat and high aniline point oils.

Preferably, the system is configured for feeding gas and/or liquid into the sampling chamber, in particular via one or more ports. Preferably, the sampling head has one or more ports for feeding gas and/or liquid into the sampling chamber and/or for discharging the gas and/or liquid from the sampling chamber. The same port can be used for inputting and outputting the same gas or liquid. In preferred embodiments, an inlet and outlet is provided for gas feed and gas discharge in/from the sampling chamber and an inlet and outlet for liquid feed and liquid discharge in/from the sampling chamber, the liquid preferably being an electrolyte composition.

Preferably, the system is configured for feeding gas and/or liquid into the second chamber, in particular via one or more ports. Preferably, one or more ports for feeding gas and/or liquid into the second chamber and/or for discharging the gas and/or liquid from the second chamber. Preferably, the second chamber is provided with at least a port for feeding and/or discharging liquid into the second chamber, in particular a liquid composition. The system may be configured for inputting and outputting liquid into the second chamber in a batchwise manner (e.g. via one port or via an inlet and outlet or in a continuous manner (e.g. via an inlet and outlet).

More preferably, the gas and/or liquid ports of the sampling chamber comprise an inlet port for feeding gas and/or liquid into the sampling chamber and an outlet port for discharging the gas and/or the liquid out of the sampling chamber. In this manner, reaction conditions within the sampling chamber can be adjusted by inputting a predetermined gas and/or liquid. Furthermore, reaction conditions or parameters can be tested by outputting the gas and/or liquid after or during the reaction within the sampling chamber, in particular if the outputted gas and/or liquid is subjected to an analysis, more in particular an analysis chosen from chromatography (CG), mass spectrometry (MS), spectroscopy, such as UV-Vis spectroscopy, Nuclear Magnetic Resonance (NMR), electrochemical analysis, titrations, and the like.

Preferably, the sampling chamber has one or more liquid ports for feeding liquid, preferably a liquid electrolyte composition, into the sampling chamber, preferably via an inlet port, and for discharging liquid out of the sampling chamber, preferably via an outlet port. Herein, by referring to "inlet" and "outlet", it is to be understood that these ports are separate ports distinguishable from each other. The overall operation of the system may be improved by having separate ports.

Preferably, the sampling chamber has at least one inlet/outlet port that is a gas port arranged to allow analysis of gas delivered and/or gas formed in the headspace of the sampling chamber. This way, the testing of the reaction conditions can be improved. The gas can be delivered before the reaction occurs, or during the reaction, and/or gas may also be formed during the reaction. More specifically, the headspace as used in this context denotes an area in the upper half of the sampling chamber.

Preferably, the system is configured to feed the sampling chamber with a gas composition via a gas inlet port, preferably for saturating an electrolyte composition within the sampling chamber. In this manner, influence of gas on the reaction within the sampling chamber can be initiated and measured.

Preferably, the sampling chamber has at least one gas inlet port arranged at or near the bottom of the chamber such that, when the chamber is filled with liquid, that the gas is delivered through said liquid, for example in a bubbling manner. In this way, the liquid within the sampling chamber may be saturated with a gas, in particular comprising or consisting of CO₂. More specifically, the arrangement of the gas inlet port at or near the bottom indicates an arrangement in a lower half of the sampling chamber.

Preferably, the system further has a gas reservoir for delivering gas into the sampling chamber via a gas inlet port of the sampling chamber or is configured to receive gas from an external gas reservoir, such as a gas cylinder, and to deliver said gas into the sampling chamber via a gas inlet port. The system may for example be embodied within a device and the gas reservoir may be incorporated in the device (e.g. as an internal reservoir) or the device may be configured to receive gas from an external reservoir, in particular via a gas inlet connection.

Preferably, the system is configured to feed the sampling chamber and/or the second chamber with an electrolyte composition via a first inlet port. The filling may be regulated with a control unit that is configured for regulating the input (e.g. flow rate, volume) of electrolyte composition into the sampling chamber and/or the second chamber. Preferably, each chamber has as inlet and an outlet for electrolyte feed and discharge.

Preferably, the system further comprises a first liquid reservoir for delivering liquid to the sampling chamber via a liquid inlet port of the sampling chamber and/or further comprising a second liquid reservoir for delivering liquid to the second chamber via a liquid inlet port of the second chamber. By having multiple liquid reservoirs, the overall functionality and performance of the system can be improved. More preferably, at least one liquid reservoir is reserved for an electrolyte composition while another one can contain a cleaning liquid for cleaning the sampling chamber, in particular after the reaction has occurred.

Preferably, one or more sensing probes are arranged within the sampling chamber for in-situ probing reagents and/or reaction conditions within the sampling chamber. In this manner, the behavior of the electrochemical reaction can be tested in an improved manner since more information can be gathered from the sampling chamber, preferably in a real time manner.

Preferably, one or more sensing probes are arranged within the second chamber for in-situ probing to receive input on conditions within the second chamber. In this manner, more information can be gathered from the second chamber. The sensing probes could encompass various instruments or devices designed to measure different parameters or properties. For instance, these probes might include sensors for measuring temperature, pressure, chemical composition, pH levels, concentration, conductivity.

Preferably, the sample holder includes a conductive path that is configured to provide an electrical connection between sample materials held by the sample holder and a power supply. In this manner, a potential can be supplied to a sample material to be tested via the conductive path, in particular to initiate a reaction within the sampling chamber. Preferably, the power supply is a potentiostat, such that a more precise control and application of potential can be applied. Preferably, the system is provided with a potentiostat to control the potential between the working electrode and the reference electrode.

More preferably, the sample holder can include several conductive paths that are electrically separated or separable from each other to allow individual application of current or potential at a preselected sample of the sample materials. The conductive paths can be made electrically separable via one or more switches.

Preferably, the system further comprises a feed-controller, for controlling gas and/or liquid input into the sampling chamber. The feed controller can enable automation, allowing for consistent and reliable control without the need for constant manual adjustment. This is particularly useful in continuous processes to test the electrochemical reaction or potential of the sample materials, in particular potentially useful catalysts.

Preferably, the system further comprises one or more analyzers, such as an autosampler, configured to receive the gas and/or the liquid from the sampling chamber and to perform an analysis thereon. For example, the analyzer may be configured to receive the gas and/or the liquid via an outlet of the sampling chamber. More in particular, the analyzer is preferably configured to perform an analysis that is chosen from a Gas or Liquid Chromatography analysis, a Mass Spectrometry analysis, a (ultra) high performance liquid chromatography (HPLC) analysis, a Nuclear Magnetic Resonance (NMR) analysis, an Inductively Coupled Plasma (ICP) analysis. In this way, more information on the reaction at the sample material within the sampling chamber can be achieved which benefits the understanding thereof.

Notably, the system may equally be configured for delivering gas and/or liquid to one or more external analyzers.

Preferably, the sample holder is configured for holding the sample materials separately such that predetermined sample materials are individually presentable to the sampling head. In this manner, individual testing can be performed to get data on each individual sample. According to a preferred embodiment, the system is configured to test individual samples in sequence, for example one after another. Multiple sampling heads may also be present within the system, such that samples can be tested in a parallel fashion, e.g. two at the same time. This may reduce overall processing time to test a large number of test samples. Herein, the terms "samples", "test samples" and "sample materials" are used interchangeably.

More in particular, the system may be a system for testing sample materials for electrocatalytic reactions in a parallel manner, said system comprising
a sample holder for holding the sample materials to be tested, and
a first sampling head and second sampling head for testing the sample materials, wherein each sampling head of the first and second sampling heads comprises an allocated individual sampling chamber separated from an allocated individual or shared second chamber, wherein the allocated sampling chambers are separated via a membrane separator from the allocated individual or shared second chamber; wherein the sample holder is configured to operate as a working electrode; wherein the sampling chamber comprises a reference electrode (RE); wherein the second chamber(s) comprise a counter electrode; and wherein the system includes a displacement arrangement for moving the first and second sampling head and/or sample holder relative to the first and second sampling head and into a testing position.

In said parallel working system, the second chamber may be shared between the first and second sampling heads, for example the first sampling head may have a first sampling chamber and the second sampling head may have a second sampling chamber second wherein both the first and second sampling chamber are separated from the same second chamber (shared second chamber system) via a membrane, in particular their own allocated membrane. In another embodiment (non-shared second chamber system), the first sampling head has a sampling chamber and a second chamber (also described herein as "reference chamber") and the second sampling head has a sampling chamber and a second chamber. Hence, in this exemplary embodiment there are at least four chambers in total since each of the first and second head has two chambers. Having multiple sampling heads benefits processing time and testing efficiency. The system may thus comprise a second sampling head, a third, and so forth, wherein said multiple sampling heads are configured to work in parallel to the first sampling head. Preferably, the system is configured to analyze electrocatalytic reactions of the sample material in series or in parallel by way of a second sampling head working in parallel to the first sampling head. In case of a second sampling head working in parallel, each of the first and second head may have their own sampling chamber and second chamber or each of the first and second head may have their own sampling chamber and share a second chamber.

Preferably the sample holder is configured for holding the sample materials separately such that predetermined sample materials are individually presentable to the sampling head. In an embodiment, the sample holder is configured to directly receive and hold the sample materials or is configured to receive the sample materials indirectly. More in particular, the sample holder is preferably configured to receive and hold a removable holding structure, such as a wafer. In this case, the sample holder indirectly holds the samples via the removable holding structure. Due to the removable holding structure, the sample materials can be arranged or placed easily on the holding structure from outside the system, for example sample materials can firstly be arranged on the wafer as a coating and can then be placed within the system whereby the sample holder firmly holds the wafer with the sample materials. After performing the test measurements, the wafer with the sample materials can be removed again from the system.

According to an embodiment, the sample holding plate is rotationally arranged. This arrangement may provide more flexibility and/or displacement options. In this manner, more options can be available to present the samples to the sampling head. For example, the samples and/or the removable structure holding the samples can be rotated, e.g. by 90°.

Preferably, the system is configured to perform test measurements of the sample materials in an automated manner. In this manner, processing time and/or people input can be reduced. Also, higher numbers of potentially useful catalytic sample materials can be tested more efficiently and rapidly.

Preferably, the sampling chamber is closable so as to close of the sampling chamber from the environment. The sampling chamber may be provided with a control valve to regulate pressure within the sampling chamber. The sampling chamber is preferably provided with a closing system which in closed condition shields the inside of the sampling chamber from the environment. In this manner, test conditions are better maintained so as to avoid influence from the outside which may impact test results.

A preferred aspect relates to the use of the system as described herein, in an analysis of electrocatalytic reactions of the sample materials, in particular wherein said analysis is performed in series by one sampling head or in parallel by way of a first and second sampling head working in parallel. Namely, electrocatalyst research is typically carried out on the lab scale under well-controlled conditions, while a variety of different parameters may influence the electrocatalyst performance e.g., applied potential, electrocatalyst elemental composition, electrocatalyst morphology (particle shape, size, crystal structure), electrolyte composition, pH, solvent (protic or aprotic), etc. By using the system as described, discovery and development of new electrocatalysts is facilitated, in particular at a higher throughput by way of the displacement arrangement.

### Brief description of drawings

The invention will now be described in more detail with respect to the drawings illustrating some preferred embodiments of the invention.
**Figure 1** shows a system for testing sample materials for electrocatalytic reactions.
**Figure 2** illustrates a perspective view of a sample holder and sampling head.
**Figure 3** illustrates a cross sectional view of a sample holder and a sampling head.
**Figures 4A** - **4B** schematically illustrate systems having displacement arrangements according to exemplary embodiments.
**Figures 5A - 5C** illustrate systems whereby the sample head is in a test position, figure 5B shows that the holder is directly holding the samples, figure 5C shows that the holder is indirectly holding the samples.
**Figures 6A** - **6B** illustrate example of systems with a sample head including multiple liquid and gas ports.
**Figures 7A** - **7B** illustrate examples of systems having multiple sampling chambers.
**Figures 8A** - **8B** **illustrate** an example of a removable holding structure for holding the samples, whereby figure 8A shows a perspective view and figure 8B shows a cross section.

In the figures the elements denoted by identical reference numbers are intended to signify components that are identical or akin in functionality and purpose. These elements may thus provide for analogous or similar technical advantages.

### Detailed Description

An aspect of the invention provides a system for testing sample materials for electrocatalytic reactions. Electrocatalytic reactions involve the use of catalysts to facilitate and expedite specific electrochemical reactions. These reactions typically occur at the interface between an electrode and an electrolyte solution. Catalysts can play a crucial role in increasing the rate of an electrochemical process and in steering the product selectivity. In view of the (growing) importance of catalysts within several industries, there is a need for researching potential catalysts. As a result, there is a need to conduct tests on multiple materials to assess their potential catalytic effects and observe their behavior throughout an electrocatalytic reaction.

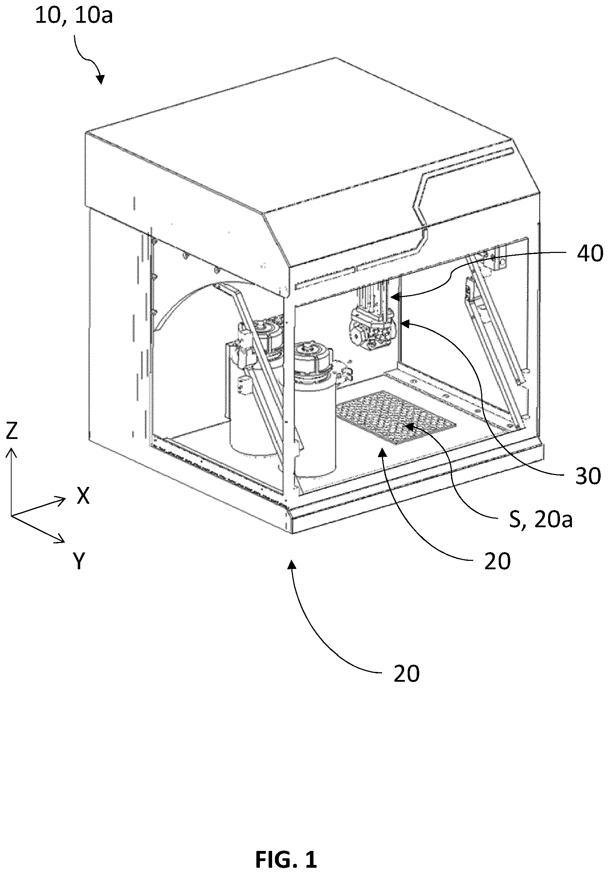

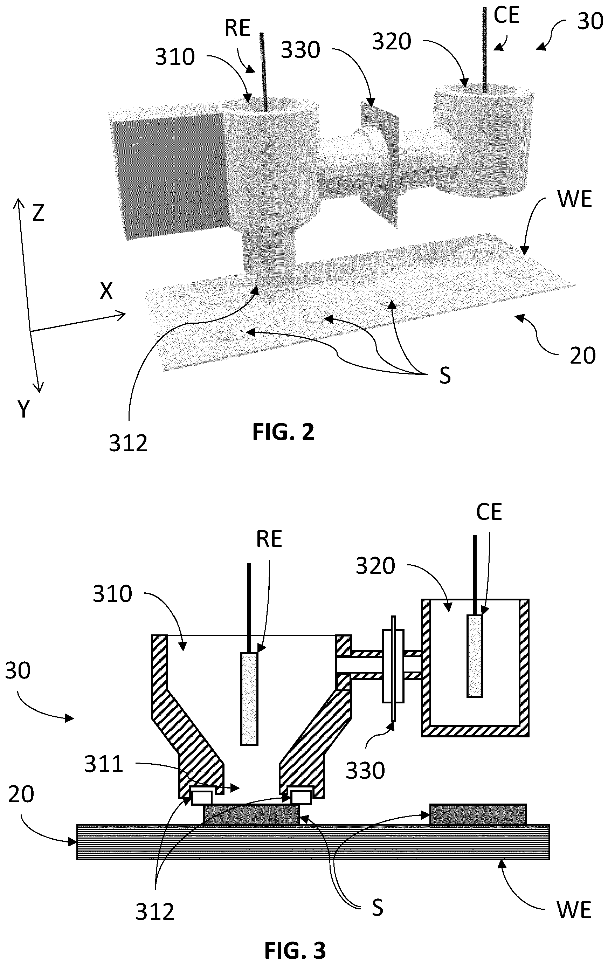

**Figure 1** shows a system 10 for testing sample materials for electrocatalytic reactions. More in particular, figure 1 shows a system 10 that is embodied in a testing device 10a having a sample holder 20 that is holding the sample materials S to be tested via the sampling head 30. The sampling head 30 has a sampling chamber (as described more in detail further) and a second chamber (as described more in detail further). Indeed, the features of these chambers will be explained with more detail in connection to figure 2 - 7.

The device 10a can automatically test the samples S via displacement arrangement 40 that is configured to move the sampling head 30 above the sampling sample holder 20 in a test position.

In figure 1, the displacement arrangement 40 is configured for moving the sampling head 30 in three dimensions: X-axis (left to right), Y-axis (front to back), and Z-axis (up and down). These movements can be controlled by an actuator system, for example an actuator system comprising electrical motors, said actuator system preferably being configured to operate based on software instructions.

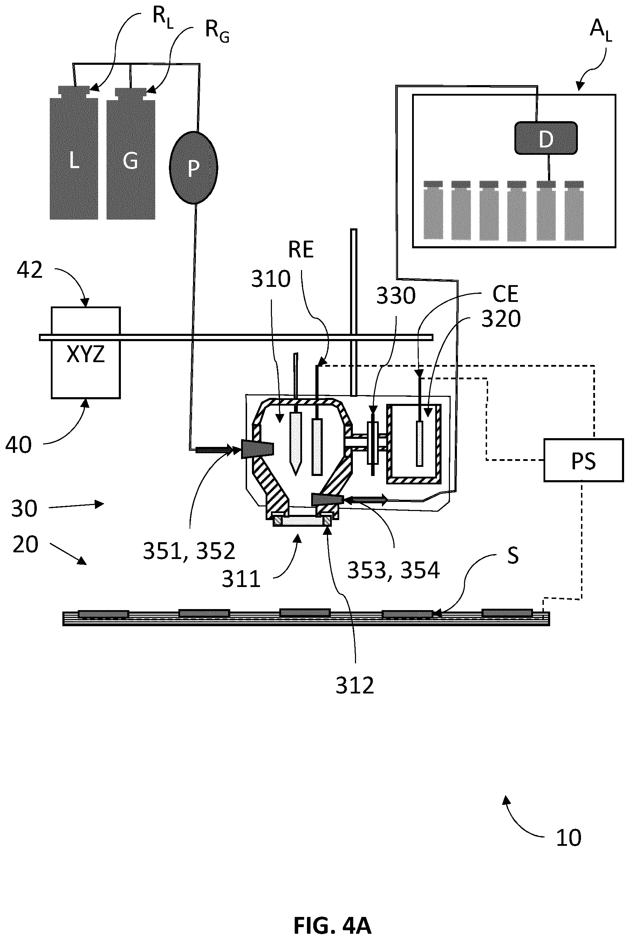

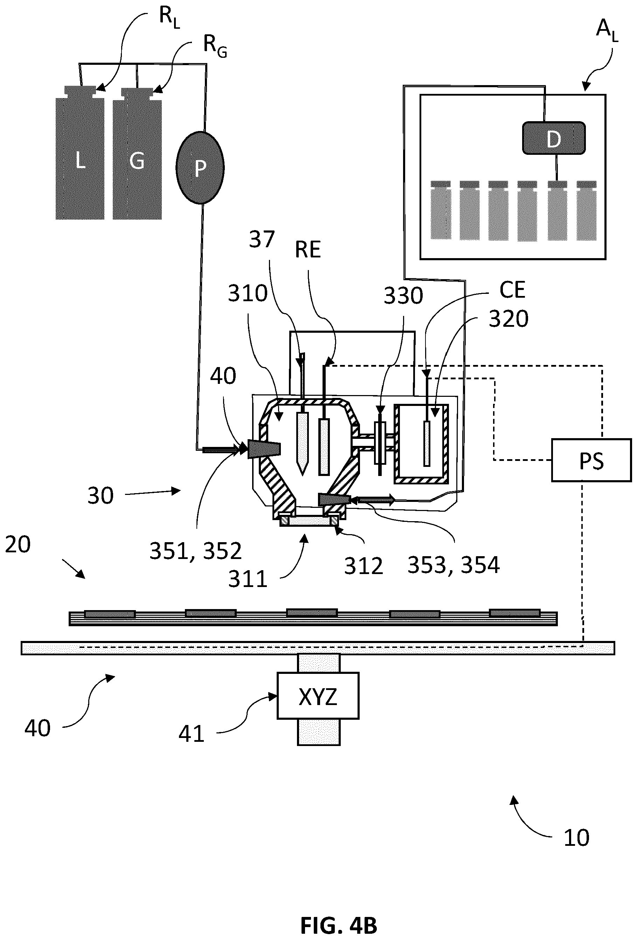

Notably, any suitable displacement arrangement 40 can be used so as to move the sample holder 20 and/or the sampling head 30 relative to each other in order to achieve a testing position such that the sample materials can be tested. For example, the sample holder can be configured as a movable sample holder that can be moved with respect to the sampling head for positioning the sample materials (see figure 4B). In addition, or as an alternative, the sampling head can be moved (see figure 4A). The displacement arrangement is preferably operatively connected to a control unit (not shown) such that the displacements can be controlled in an automated manner.

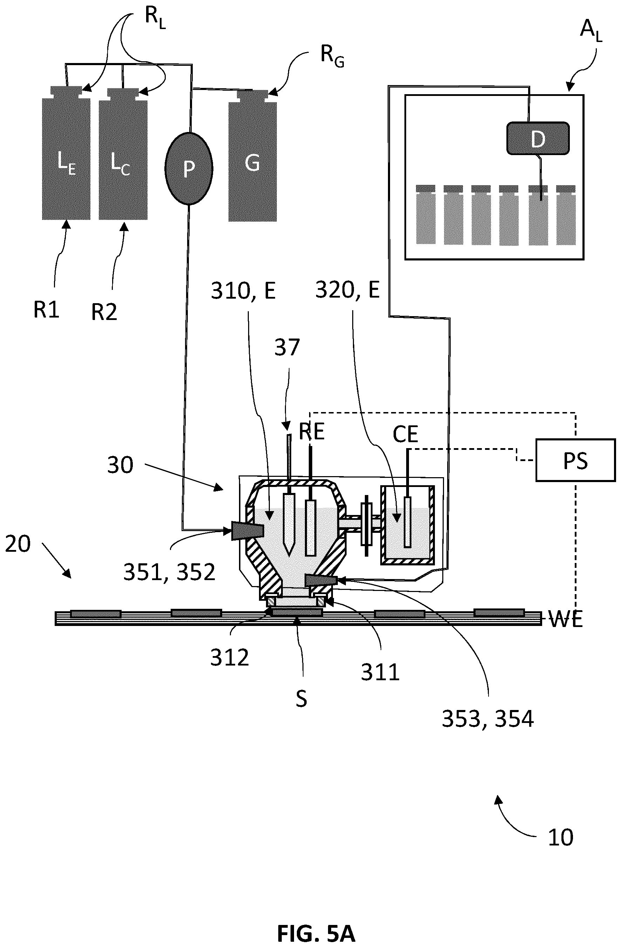

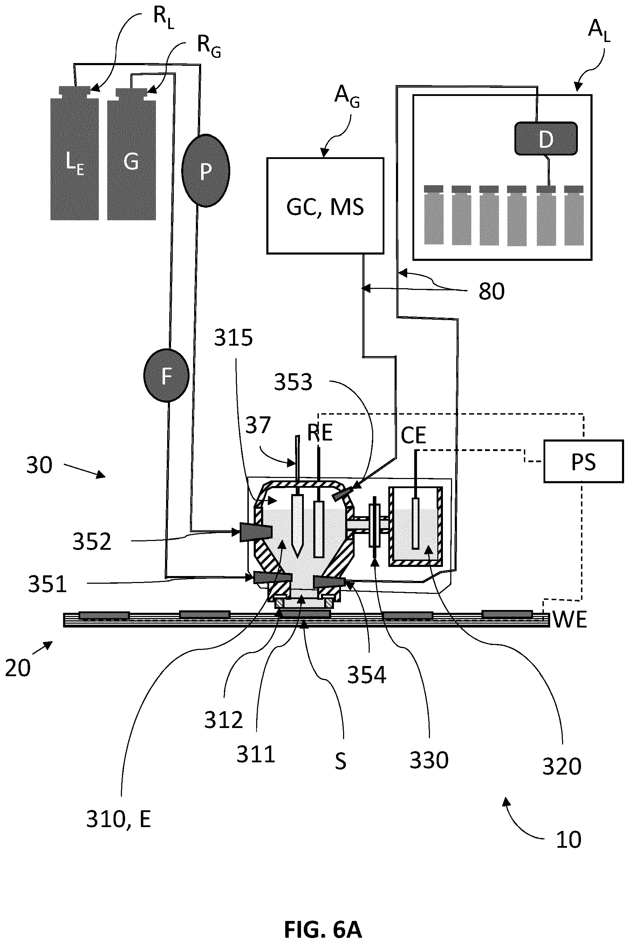

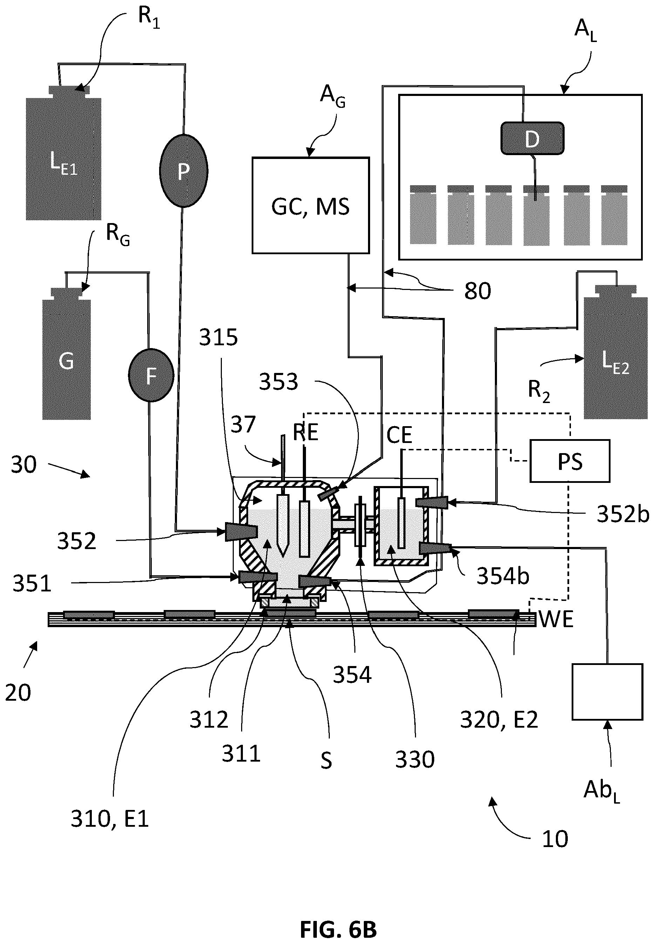

In device 10a as shown in figure 1, the sample holder 20 is configured to receive and hold a removable holding structure 20a. In particular, the sample holder 20 is configured to hold the removable holding structure 20a such that the holding structure stays in place at a predetermined position during testing. An example of a removable holding structure 20a will be explained in connection with figure 6. The sample holder 20 may be provided with clamps or pins (not shown) to engage and hold the removable structure in place during testing. By using a removable structure, the sample materials S can be arranged or placed easily on the holding structure 20a before being installed on the sample holder 20 within device 10a. The sample holder 20 as shown in figure 1 can have any of the features as described further (see e.g. the holder 20 as described in connection to figures 5A-5C).

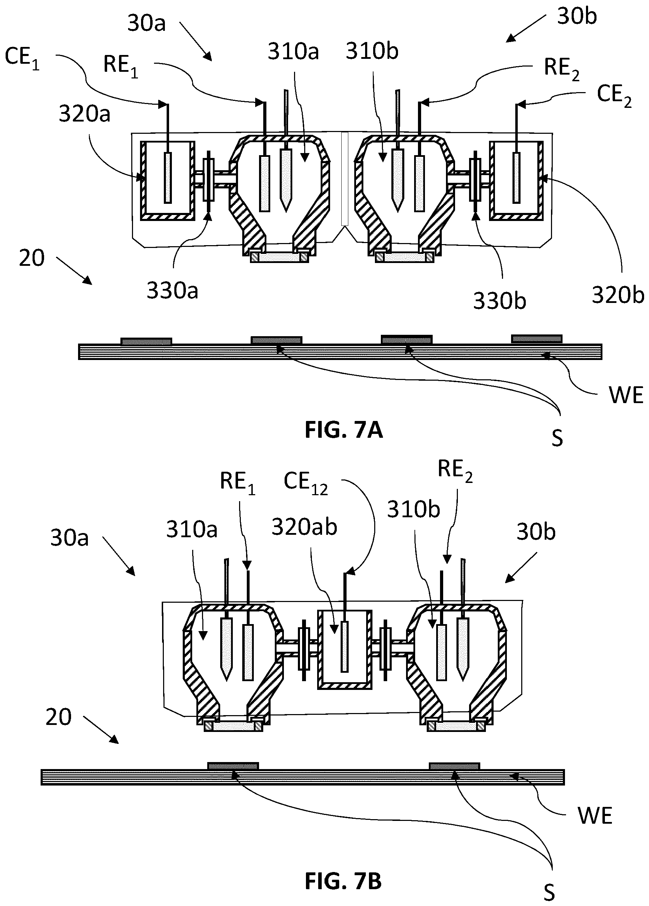

Understandably, the system 10 may be configured to fill the second chamber 320 (Fig 7A) with electrolyte and to discharge the second chamber 320. In particular, the second chamber 320 may include one or more ports (not shown in fig. 1) for feeding/discharging electrolyte composition from said chamber 320, as will be explained further in connection to Figure 6A.

Generally applicable herein, the feeding and/or discharging of gas and/or liquid may be controlled by a feed/discharge controller.

Also the second chamber 320 may be provided with any gas outlets (not shown) configured to discharge the gas, more in particular the gas outlets can be arranged to discharge and deliver gas from the second chamber 320 to a gas analyzer.

**Figures 2 and figure 3** shall now be used to further explain principles of the sampling head 30. The sampling head 30 has sampling chamber 310 that is separated from a second chamber 320 via a membrane separator 330. The membrane separator 330 is preferably an ion exchange membrane (e.g. anion exchange membrane, cation exchange membrane or bipolar membrane) or a gas separator membrane (e.g. zirfon). The sampling chamber 310 of sampling head 30 has a reference electrode RE. The second chamber 320 has a counter electrode CE.

The sample holder 20 is configured to operate as a working electrode WE for applied samples S, more in particular configured such that a potential or current can be applied to the samples S when held by the sample holder 20, either directly or indirectly via a removable holding structure 20a. The potential / current may be applied to the samples S, preferably by aid of a potentiostat, by applying the potential/current to the sample holder 20 and/or to the removable holding structure 20a.

A working electrode WE in an electrochemical system typically refers to an electrically conducting surface where the electrochemical reaction occurs. In the present instance, corresponding to the surface of the sample(s) S being tested.

The reference electrode RE is typically used to provide a stable and known electrical potential against which the potential of the working electrode can be measured. Materials like Silver/Silver Chloride (Ag/AgCl) or mercury-mercurous chloride (Hg/Hg₂Cl₂) can be used for the reference electrode.

The counter electrode CE is preferably used to complete the circuit in the electrochemical cell during testing. In this manner, the counter electrode CE balances electron flow generated at the working electrode by either supplying or accepting electrons to maintain charge neutrality. Preferably, the counter electrode is configured to not participate in the reactions occurring at the working electrode to ensure accurate measurements. Materials like platinum or graphite are preferably used as counter electrodes due to their inert nature. More preferably, a high surface area metal such as nickel foam, titanium felt, platinum/tantalum and/or a dimensionally stable anode (DSA) is used for the counter electrode.

As shown in figure 3, the sampling chamber 310 has a sample opening 311 which is placeable on or over a sample S on the sample holder 20 that is configured to work or operate as the working electrode WE, in particular by aid of the displacement arrangement (not illustrated in figures 2 and 3). In this manner, the sampling head can be placed automatically in a test position.

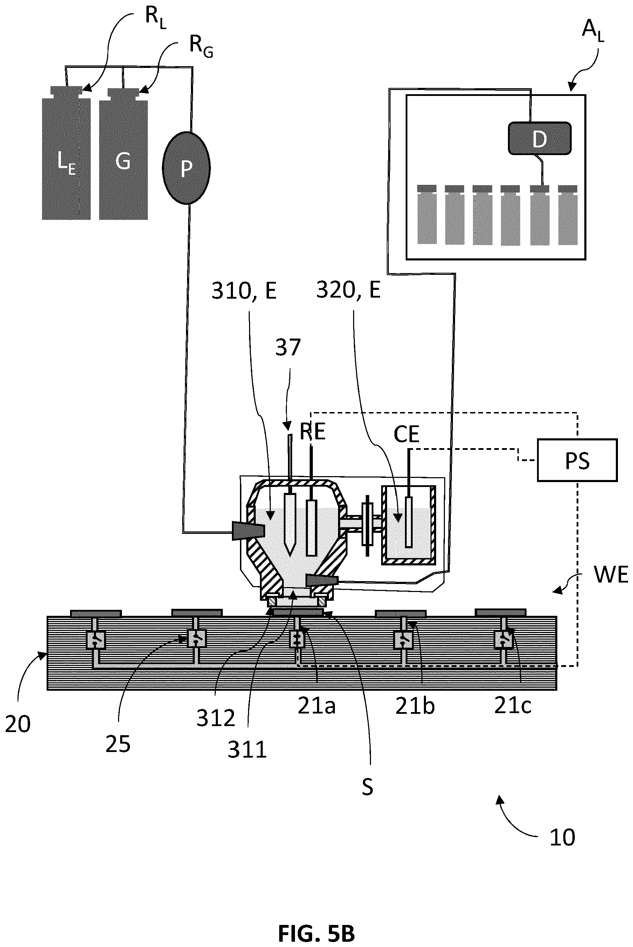

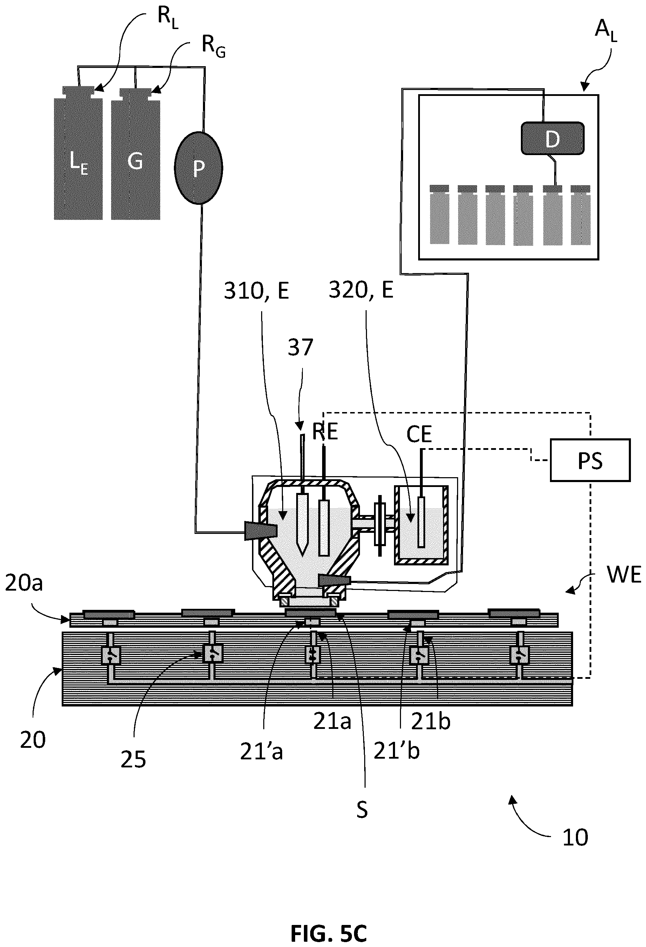

As said, the sample holder 20 may directly hold the samples S or indirectly, for example the samples S can be arranged on a conductive plate 20a before being held by the sample holder 20. To improve operation and reduce risk of electrolyte E (see fig. 5) leakage, the opening 311 is preferably provided with a sealing ring 312 that is arranged to seal off the sampling chamber 310 when the sample opening 311 is placed on or over a sample S on the sample holder 20.

Figure 4A and figure 4B shall now be used to explain preferred displacement arrangements 40 (see actuator 42 and positioning table 41). The head 30 can be moved towards the sample holder 20 or the other way around, a combination is also possible.

**Figure 4A** illustrates that the displacement arrangement 40 is configured for displacing the sampling head 30 and to move said sampling head 30 into test position, in particular a position above the sample holder 20. More specifically, figure 4A shows that displacement arrangement 40 is provided with an actuator 42 to position the sampling head 30 into a test position. The displacement arrangement 40 can include one or more actuators to displace the sampling head 30 in any one of the XYZ directions, or a combination thereof, as desired.

**Figure 4B** illustrates that the displacement arrangement 40 is configured for displacing the sample holder 20 and to move said sample holder 20 into a test position, in particular a test position under the sampling head 30. Any one of the holder 20 and head 30 can be arranged fixedly as long as the displacement arrangement 40 can displace the other component into test position. For example, the head 30 can be arranged fixedly and the holder 20 can be displaced via positioning stage 41 (figure 4B). Also, for example, the holder 20 can be arranged fixedly and the head can be displaced by one or more actuators 20. It is also possible that both the holder 20 and the head 30 are both movably arranged such that both components can be displaced by displacement arrangement.

Understandably, as used herein "displacement" or "displacing" can include any XZY-movements, any rotational movements and any combination thereof. For example, the head 20 may be movable in a Z direction (up and down) while the holder 20 can be rotated via a movable and/or rotatable table 41.

Figure 4A and figure 4B further illustrate that the sampling head 30 can have one or more ports (see for example gas inlet 351, liquid inlet 352, gas outlet 353, liquid outlet 354 for feeding gas G and/or liquid L into the sampling chamber 330 and/or for discharging the gas G and/or liquid L from the sampling chamber 330. Depending on the process, the feed and discharge of gas G or liquid L may be done in a continuous-manner or in a batch-wise manner.

Preferably, at least one gas outlet 353 is arranged in an upper part of the sampling chamber 310 (see Figure 6A) such that gas can be discharged from the headspace 315 of the sampling chamber 310, more preferably the discharged gas delivered to an analyzer.

As illustrated in figure 4A, the gas and/or liquid may be fed via a shared inlet port (see 351, 352) and discharged a shared outlet port (see 353, 354). Preferably, as illustrated in Figure 6A, the gas and liquid are fed and discharged via a designated inlet port and outlet port. Preferably, gas is fed via gas inlet port 351 and discharged via gas outlet port 353. Preferably, liquid L is fed via inlet port 352 and discharged via outlet port 354.

The system may include one or more gas/liquid feed reservoirs (see reservoirs L and G) and one or more discharge gas/liquid reservoirs or may be configured to interact therewith, e.g. to receive gas or liquid therefrom. More specifically, the system may be configured to receive gas and/or liquid from external reservoirs.

In particular, the system 10 preferably comprises a liquid reservoir L for feeding the electrolyte composition E into the sampling chamber 310 and/or the system 10 preferably comprises a gas inlet 351 for feeding gas into the sampling chamber 310 and a gas outlet 353 for discharging gas after testing.

Preferably, as e.g. illustrated by Figure 6A, the system 10 is configured to analyze gas discharged from the sampling chamber 310, in particular by aid of a gas analyzer A_{G}, more in particular a gas analyzer A_{G} chosen from an analyzer that is configured to perform an electrochemical testing, a gas chromatography testing, a mass spectrometry testing or combinations thereof on the gas that is discharged from the sampling chamber 310.

The analyzer may be part of the system or the system can be configured to communicate therewith, for example via one or more outlet ports and/or via one or more tubes or conduits 80. In particular the system is preferably configured to deliver gas and/or liquid from sampling chamber 310 to one or more gas/liquid analyzers (see A_{G}, A_{L}) which can be internally or externally arranged. Figure 6A illustrates that sampling chamber 310 has a gas outlet 353 for discharging gas from the sampling chamber 310 and to deliver said discharged gas to the gas analyzer A_{G}, for example via conduit 80. Figure 6A further illustrates that liquid outlet 354 is connected or can be connected to liquid analyzer A_{L}.

Although not shown, it is further understandable that the system may be configured to bring gas/liquid from the second chamber 320 to one or more analyzers in a similar manner.

Preferably, the sampling chamber 310 has a gas outlet 353 which is connectable or is connected to a gas analyzer A_{G} (see Figure 6A) for analyzing the gas fed of formed in the sampling chamber 310.

Preferably, the sampling chamber 310 has a liquid outlet 354 which is connectable or is connected to a liquid analyzer A_{L} (see Figure 6A) for analyzing the electrolyte composition E discharged from the sampling chamber 310.

Preferably, the system 10 is configured for feeding and discharging a cleaning liquid into the sampling chamber 310 and/or into the second chamber 320, for example between the testing of different samples. Therefore, according to preferred embodiments, the system 10 may include an internal reservoir L for feeding the cleaning liquid into the sampling chamber 310 and/or into the second chamber 320.

**Figure 5A** shall now be used to explain how the system 10 can be used to test sample materials S. The sampling head 30 is placed into testing position by aid of a displacement arrangement (not shown in fig. 5A, see XYZ displacement arrangement 40 in figure 4A and 4B) such that the opening 311 of the sampling chamber 310 is placed on sample S on the sample holder 20.

The sample chamber 310 (RE-chamber) can be filled with electrolyte E so as to form an electrical connection between the reference electrode RE and the sample S on the sample holder 20 that is configured to operate as the working electrode WE either directly or via a removable sample holding structure 20a.

The sample holder 20 can be configured to provide a conductive path between a power source PS (preferably a potentiostat) and the sample S so as to apply a potential on the sample S with respect to the RE in order to induce an electrochemical reaction within the sampling chamber 310 and test the electrochemical behavior of the sample S. In case the sample holder 20 comprises a removable sample holding structure 20a, the power source PS can be connected to the removable sample holding structure 20a directly or via the sample holder 20.

The sample holder 20 is preferably conductive and/or includes conductive paths arranged throughout the holder for connecting a power source directly to samples on the sample holder or indirectly via an intermediate holder, such as a sample array (see intermediate holder 20a in figure 5C). The sample holder 20 is preferably configured to receive a sample array 20a (as a removable structure), wherein said sample array 20a is electrically conducting such that it allows an electrical connection between various sample materials placed on the array and a power supply, preferably a potentiostat PS.

Figure 5A further shows that the system 10 may include reservoirs (see L_{E} and L_{C}) for liquid feed into the sampling chamber 310, preferably a reservoir L_{E} for feeding the electrolyte composition and optionally a reservoir L_{C} for a cleaning liquid to clean the inside of the sampling chamber 310 between testing of several samples S. The feeding of the liquid may be controlled by a feed-controller P for controlling liquid input into the sampling chamber 310. In addition, the feeding of gas may be controlled by the same controller P or a different controller P.

As shown in figure 5A, the sealing ring 312 facilitates the pressing and sealing of the sampling head 30 onto the sample S, more specifically the sealing ring 312 avoids leakage of the electrolyte composition E.

The sealing ring 312 is preferably a pressable material, more preferably a synthetic rubber, in particular a material chosen from Ethylene Propylene Diene Monomer (EPDM), Nitrile Butadiene Rubber (NBR), FKM (Fluoroelastomer, Viton), Silicone. Generally preferred herein, the ring should be chemically resistant to the electrolyte E composition. The material of the X-ring should be chemically resistant depending on the electrolyte or solvents to be used as well as on the reaction conditions such as temperature. In the case of an aqueous neutral/alkaline electrolytes e.g. KHCO₃, NaHCO₃, phosphate buffer, KCI, NaCl, KOH, NaOH solutions, Ethylene Propylene Diene Monomer (EPDM) can be used. Furthermore, it is suggested to use the sealing material with the hardness of 70-85 Shore A.

Preferably, the system is configured to interact with a power source, preferably a potentiostat to regulate the application of electrical potential / current on the samples. Exemplary electrical pathways are indicated via dashed lines in the figures (see figure 4A to Figure 6A).

Further information (e.g. reaction conditions, including temperature, pH, conductivity) from within the sampling chamber 310 can be gathered via sensing probe 37. Preferably, at least one sensing probe 37 is arranged to sense reaction conditions within sampling chamber 310.

Figure 5A further indicates that the system may further have a first liquid reservoir R1 for delivering liquid, preferably an electrolyte liquid, to the sampling chamber 310 via a liquid inlet port 352 of the sampling chamber 310 and a another liquid reservoir R2 for delivering a second liquid into the sampling chamber 310 (as shown) or the second liquid reservoir R2 may be arranged to deliver a liquid into the second chamber 320, as explained further in connection to figure 6B.

Indeed, the second chamber 320 may also comprise one or more ports (not shown), in particular a liquid feed port (not shown) for feeding liquid, in particular an electrolyte composition, into the second chamber 320.

It is generally desired herein that the system is configured to fill the sampling chamber 310 with an electrolyte composition E via a first inlet port 351 of the sampling chamber 310. The sampling chamber (RE-chamber) may be filled and emptied in a continuous manner or in a batch manner.

Preferably, the system is configured to fill the second chamber 320 (CE-chamber) with electrolyte composition E. The second chamber 320 may be filled via an inlet port 352b (as explained in the context of figure 6B). The second chamber 320 may be filled and emptied in a continuous manner or in a batch manner.

As generally applicable herein, the system may be provided with one or more liquid feed and/or liquid discharge controllers to regulate feed and/or discharge of liquid in and/or from the either one or both of the sampling chamber 310 (RE-chamber) or the second chamber 320 (CE-chamber).

**Figure 5B** illustrates an example wherein the sample head 30 is positioned in a test position. In the test position as shown, electrical contact between the sample S to be tested and the reference electrode RE and/or the counter electrode CE is allowed when the chambers are filled with electrolyte composition E. An example of an electrical circuit with electrical potential supplied by power source PS is indicated via the dashed lines.

In the embodiment shown in figure 5B, the sample holder 21 includes a conductive path 21 that is configured to provide an electrical connection between sample material(s) S held by the sample holder 20 and power supply PS.

Even more in particular, figure 5B illustrates that sample holder 20 includes several conductive paths (see 21a, 21b, 22c) that are electrically separable, for example via one or more switches 25 as shown. In this manner, individual application of current or potential to a preselected sample S of the sample materials is allowed. It is further possible (see figure 5C) that the sample holder 20 may be configured to hold the samples via a removable sample holding structure 20a. In such a case, the sample holder 20 comprises a removable sample holding structure 20a, e.g. a wafer, that preferably includes several conductive paths (see 21'a and 21'b) that are electrically separated from each other to allow individual application of current or potential at a preselected sample S of the sample materials when placed on the sample holder 20 such that there is an electrical connection between 21'a and 21a and between 21'b and 21b.

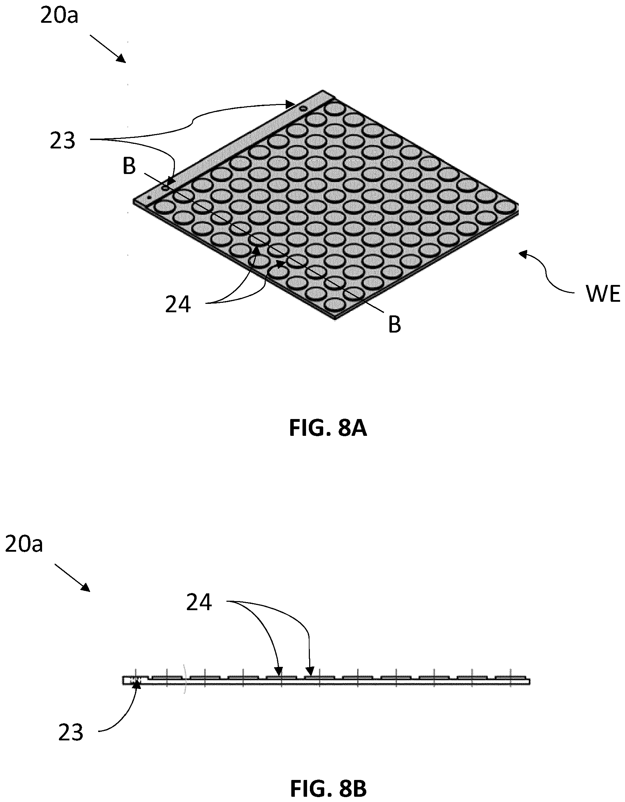

**Figure 5C** illustrates that the system comprises a sample holder 20 that is configured to (indirectly) hold the samples via the removable sample holding structure 20a. Preferably, the sample holder 20 comprises engaging means (not shown in figure 5C, further explained with figure 8) to engage and hold the sample holding structure 20a. The engaging means may be chosen from one or more clamps or may comprise pins or holes configured to engage with holes or pins of the removable holding structure 20a.

Although not illustrated by figure 5C, it is generally preferred that the sample holder 20 and holding structure 20a are provided with a positioning system configured to position and hold the removable holding structure 20a in place during testing. In an embodiment, the positioning system is arranged such that one of or both the holder 20 and holding structure 20a comprises pins that are arranged to engage with corresponding holes 23 of the other (see for example figures 8A-8B indicating holes 23). In this manner the removable sample holding structure 20a can be firmly held in place during testing by aid of said positioning system. In addition, or as an alternative one or more clamps (not shown) can be used.

As shown in figure 5C, the conductive pathways of the sample holding structure 20a and the sample holding structure 20a overlap to allow an electrical connection when the removable holding structure 20a is placed on the sample holder 20. The system is preferably configured to selectively apply a current or a potential via the conductive paths (see 21'a or 21'b) that are electrically separated.

The sample holder 20 is preferably configured for holding the sample materials S separately such that predetermined sample materials S are individually presentable to the sampling head 30 as for example shown in figures 1 to 7.

**Figure 6A** **and** **figure 6B** further show preferred embodiments of systems to test sample materials S. The samples can be held directly by sample holder 20 (as shown) or indirectly via a removable holding structure 20a (as explained earlier).

As shown by figures 6A-6B, each shown system has a sampling head 30 and a sampling holder 20 which can be moved into a test position by aid of a displacement arrangement (not shown in figure 6A) as explained earlier. The sampling chamber 310 has an opening 311 for receiving a sample S on the sample holder. In a test position, the opening 311 is placed on or over a sample S to allow an electrical connection between the sample S and the reference electrode RE via the electrolyte E when the sampling chamber 310 is filled therewith. Preferably, the opening 311 is provided with a ring 312, e.g. a rubber X-ring, to tightly press directly on the sample S and/or on the working electrode (WE) material with the sample S surrounded by the circumference of the ring. By having the ring, leakage of electrolyte can be reduced.

Figures 6A-6B further indicate that the sampling chamber 310 has at least two inlet ports 351, 352 (one port 352 for the electrolyte E inflow and one port 351 for purging reactant or inert gas), two outlet ports (one port 354 for the electrolyte outflow and one port 353 for gas discharged).

Preferably, a gas port 353 is arranged in an upper part of the sampling chamber 311 for analysis of the headspace. This gas port 353 is preferably connected or connectable to a gas analyzer A_{G} for analysis.

Figures 6A-6B further indicate that the sampling chamber 310 is connected via a channel to the second chamber 320, the channel comprising membrane separator 330 for separating the reference electrode RE and the counter electrode CE while allowing ionic conductivity via an electrolyte when the chambers 310, 320 are filled.

Figures 6A-6B further illustrate that the inlet ports 351, 352 of the sampling chamber 310 are connected to an electrolyte reservoir L_{E}, for example via tubes. Preferably, the electrolyte reservoir L_{E} is internally arranged. Further reservoirs can be included for inputting different electrolyte compositions and/or for inputting a cleaning or rinsing liquid into sampling chamber 310. The feed of liquid(s) can be regulated by feed controller P.

The gas port 351 is preferably arranged for inputting gas into a lower part of the sampling chamber 310 (as shown in Figures 6A-6B). In this manner, gas and/or reactant delivered via the gas port 351 can bubble through the electrolyte E for improved mixing and faster saturation. The gas port 351 can be connected to an internal gas reservoir G or is arranged to be connectable to an external gas reservoir. The gas feed can be regulated via gas feed controller F.

The outlet ports 353, 354 are preferably connected or configured to be connected to either HPLC or GC instruments for liquid and/or gaseous product analysis.

The sampling reaction cell defined by the internal room of the sampling chamber 310 and the second chamber 320 is preferably completely sealed to avoid undesired gas/liquid leakage or input. More in particular, the reaction cell is preferably configured to be sealable such that the reaction cell is separated from the surrounding environment during a reaction within the reaction cell. In this manner, measurement accuracy can be improved.

The sampling head 30 as illustrated in Figures 6A-6B can be mounted on a robotic arm (not shown) or any other displacement arrangement 40 as explained earlier.

Figure 6B differs from figure 6A in that it further shows that the second chamber 320 can be provided with one or more ports 352b, 354b for liquid feed and/or discharge.

As generally applicable herein, the second chamber, also called the CE-chamber, is preferably provided with one or more ports 352b, 354b for liquid feed into and/or discharge from the second chamber, in particular for feeding and discharging an electrolyte composition (indicated with E2 in figure 6B) into and from the second chamber. The electrolyte can be delivered into the second chamber 320 in a batch-wise or a continuous manner. The second chamber preferably has an inlet port 352b for feeding an electrolyte composition E2 into the second chamber and an outlet port 354b for discharging the electrolyte composition E2.

Figure 6B illustrates that the system preferably comprises a first liquid reservoir R1 and a second liquid reservoir R2. The first liquid reservoir R1 is arranged for providing a first electrolyte composition E1 into the sampling chamber 310 (also called the RE-chamber). The second liquid reservoir R2 is arranged for providing a second electrolyte composition E2 into the second chamber 320 (also called the CE-chamber). As illustrated, the second electrolyte composition E2 can be delivered into the CE chamber from reservoir R2 via inlet port 352b. The electrolyte composition E2 can be discharged via outlet 354b, e.g. to be disposed or to be analyzed by an analyzer (indicated with Ab_{L}).

The system can also comprise two or more sampling chamber 310 compartments (also called RE-compartments) and second chamber 320 compartments (also called CE-compartments) in order to do more experiments (see figures 7A and 7B) at once.

**Figure 7A** and **figure 7B** illustrate examples of systems having multiple sampling chambers (see chambers 310a, 310b). As shown, the system may be configured to analyze electrocatalytic reactions of the sample material in parallel, in particular by way of a second sampling head 30b working in parallel to the first sampling head 30a.

The first sampling head 30a may have any of the features as explained above, likewise the second sampling head 30b may have the same or similar features included analogous to those of the first sampling head.

As shown in figures 7A and 7B, the second sampling 20b head has their own sampling chamber 310a with a reference electrode RE₂, the second chamber (see chamber 320ab) of the second head 30b having the counter electrode (see counter electrode CE₁₂) may be shared (see fig. 7B) or each sampling chamber of the sampling heads may have their own second chamber (see fig. 7A, chambers 320a and 320b).

**Figure 7A** shows a non-shared second chamber system. The first sampling head 30a has a sampling chamber 310a with reference electrode RE₁ and a second chamber 320a, also called CE-chamber, with counter electrode CE₁. The second sampling head 30b has a sampling chamber 310b with reference electrode RE₂ and a (non-shared) second chamber 320b with counter electrode CE₂.

**Figure 7B** shows that the second chamber (see 320ab) may be shared between the first sampling head 30a and second sampling head 30b, for example the first sampling head may have a first sampling chamber 310a and the second sampling head 30b may have a second sampling chamber second 310b wherein both the first and second sampling chamber are separated from the same second chamber with the counter electrode (see electrode CE₁₂) via a membrane, in particular their own allocated membrane, see membrane separators 330a, 330b.

**Figures 8A** - **8B** **illustrate** a removable holding structure 20a for holding the samples. The structure 20a as shown is usable within the system as explained in connection to figure 1. More in particular, according to an aspect, there is provided a removable sample holding structure 20a for use within the system as described herein, wherein the removable sample holding structure preferably comprises a mechanically stable base layer. The mechanically stable base layer can be conductive (itself), such as a titanium or consist of a non-conductive base layer that can be modified such that a conductive path is provided on said base layer, for example by a conductive coating. The conductive character allows the application of a current or potential to samples placed on the removable holding structure 20a.

For example, a non-conductive stable base layer, such as a silicon base layer, can be coated with a conductive film or with one or more conductive strips. The non-conductive base layer may be coated with copper, such that a conductive path is provided thereof.

Figure 8A shows a perspective view of the removable holding structure 20a and figure 8B shows a cross section through section line BB. The holding structure 20a as shown is provided with holes 23 that are arranged to receive pins (not illustrated) of the sample holder 20. In this manner, the holding structure can be held firmly in place during testing by aid of the pin and holes that are functioning as a positioning system. It is understandable that any suitable positioning system can be used in order to position and firmly hold the holding structure 20a in place during testing. For example, a clamp can be used for holding structure 20a in place.

Figures 8A-8B further show that the removable holding structure 20a is provided with a plurality of raised surfaces 24 that are configured to receive samples S, more in particular a pattern of individual raised surfaces 24, even more in particular a pattern of individual raised surfaces 24 each having a substantially circular shape. Other shapes are also imaginable. Preferably, the raised surfaces have dimensions which are larger than a diameter of the opening 311 of the sampling chamber 310.

The skilled person will appreciate on the basis of the above description that the invention can be embodied in different ways and on the basis of different principles. The invention is not limited to the above described embodiments. The above described embodiments and the figures are purely illustrative and serve only to increase understanding of the invention. The invention will not therefore be limited to the embodiments described herein, but is defined in the claims.

## Claims

1. System (10) for testing sample materials for electrocatalytic reactions, said system comprising two separate components consisting of
a sample holder (20) for holding the sample materials (S) to be tested, and
a sampling head (30) for testing the sample materials, said sampling head comprising
a sampling chamber (310) separated from
a second chamber (320) via a membrane separator (330);
wherein the sample holder (20) is configured to operate as a working electrode (WE);
wherein the sampling chamber (310) comprises a reference electrode (RE);
wherein the second chamber (320) comprises a counter electrode (CE); and
wherein
the system (10) includes a displacement arrangement (40) for moving the sampling sample holder (20) and/or the sampling head (30) relative to each other and into a testing position.

2. The system of the previous claim, wherein the displacement arrangement (40) comprises
a positioning stage (41), such as a movable and/or rotatable table, configured to position the sample holder (20) and/or comprises
an actuator (42) to position the sampling head (30).

3. The system according to any of the previous claims, wherein the sampling chamber (310) comprises
a sample opening (311) configured to be placed on or over a sample on the sample holder, preferably wherein the sampling head (30) comprises a sealing ring (312) arranged to seal off the sampling chamber (310) when the sample opening (311) is placed on or over a sample on the sample holder.

4. The system according to any of the previous claims, wherein the sampling head (30) further comprises one or more ports (351, 352, 353, 354) for feeding gas and/or liquid into the sampling chamber (330) and/or for discharging the gas and/or liquid from the sampling chamber; preferably wherein the gas and/or liquid ports (351, 352, 353, 354) of the sampling chamber (30) comprise an inlet port (351, 352) for feeding gas and/or liquid into the sampling chamber (30) and an outlet port (353, 354) for discharging the gas and/or the liquid out of the sampling chamber (310).

5. The system according to any of the previous claims, wherein the sampling chamber (310) has a headspace (315) and wherein the sampling chamber (310) is provided with a gas port (353), in particular a gas outlet port (353), that is arranged to allow analysis of gas delivered and/or gas formed in the headspace (315) of the sampling chamber (310).

6. The system according to any of the previous claims, wherein the system is configured to feed the sampling chamber (310) with a gas composition (G) via a gas inlet port (351), preferably for saturating an electrolyte composition (E) within the sampling chamber, preferably wherein said gas inlet port (351) is arranged at or near the bottom of the sampling chamber (310) such that, when said chamber (310) is filled with liquid E, that the gas is delivered through said liquid.

7. The system according to any of the previous claims, further comprising a gas reservoir (G) for delivering gas into the sampling chamber via a gas inlet port (351) of the sampling chamber (310) or is configured to receive gas from an external gas reservoir and to deliver said gas into the sampling chamber (310) via a gas inlet port (351).

8. The system according to any of the previous claims, wherein the system is configured to fill the sampling chamber (310) with an electrolyte composition (E) via a first inlet port (352), preferably wherein said filling is regulated by a feed controller (P)

9. The system according to any of the previous claims, wherein one or more sensing probes (37) are arranged within the sampling chamber (310) for in-situ probing reagents and/or reaction conditions within the sampling chamber.

10. The system according to any of the previous claims, wherein the sample holder (20) includes several conductive paths (21a, 21b, 21c) that are electrically separated or separable from each other to allow individual application of current or potential at a preselected sample (S) of the sample materials (S).

11. The system according to any of the previous claims, further comprising a feed-controller (P, F), for controlling gas and/or liquid input into the sampling chamber (310).

12. The system according to any of the previous claims, further comprising an analyzer (A_{G}, A_{L}) and/or wherein the system is configured to deliver gas and/or liquid from sampling chamber (310) to an analyzer (A_{G}, A_{L}), wherein the analyzer (A_{G}, A_{L}) is configured to receive gas and/or liquid from the sampling chamber (310) and to perform an analysis thereon.

13. The system according to any of the previous claims, wherein the sample holder (20) is configured for holding the sample materials (SM) separately such that predetermined sample materials (SM) are individually presentable to the sampling head.

14. The system according to any of the previous claims, wherein the system is configured to perform test measurements of the sample materials in an automated manner.

15. The system according to any of the previous claims, wherein the system is configured to analyze electrocatalytic reactions of the sample material in series by way of the first sampling head (30, 30a) or in parallel by way of a second sampling head (30b) working in parallel to the first sampling head (30, 30a), wherein the second sampling head (30b) shares the second chamber (320ab) of the first sampling head (30a) or wherein the second sampling head (30b) comprises a further second chamber (320b).
